# EUROPEAN PATENT SPECIFICATION

(11) **EP 1 435 249 B1**
(45) Date of publication and mention of the grant of the patent: **13.05.2009**
(21) Application number: 04075016.8
(22) Date of filing: 02.01.2004
(51) Int. Cl.: A61L 24/04, A61L 24/00

(54) **Composition for bone treatment**
Zusammensetzung zur Behandlung von Knochen
Composition pour le traitement des os

(30) Priority: 02.01.2003 EP 03075001
(43) Date of publication of application: 07.07.2004
(60) Divisional application: 09155009.5
(73) Proprietor: de Vries, Alexander Cornelis, 3014 HB Rotterdam (NL)
(72) Inventor: de Vries, Alexander Cornelis, 2512 HD Den-Haag (NL); van Turnhout, Jan, 2641 CW Pijnacker (NL); van den Berg, Otto, 2651 MS Berkel en Rodenrijs (NL)
(74) Representative: Prins, Adrianus Willem

(56) References cited:
- US-A- 4 429 062
- US-B1- 6 306 177

## Description

The invention relates to a biocompatible polymer composition, suitable for *in vivo* vessel repair, for treatment of a bone.

A catheter system for delivering fluid materials, such as medicaments to a body vessel is reported in EP 0 667 131 A2. The fluid material is for example a mixture comprising an epoxy resin that cures in the presence of ions.

WO 96/182427 relates to an *in situ* stent forming catheter for delivering drugs and other fluid materials to an isolated area of a human vessel. As examples of fluid materials, mixtures comprising moisture curing polymeric materials are mentioned, such as cyanoacrylate, polymethylacrylate, polylactic acid and polyglycolic acid.

The American patent US-A 6,306,177 describes a method and a related composition for repairing tissue, in particular bone and cartilage. The method involves the use of a curable polyurethane. The publication describes curable polyurethane compositions in general terms and is silent about specific characteristics of a composition for use in (aortic) aneurism repair, such as the viscosity requirements in combination with specific required or desired physical features of the cured compositions, inside the blood vessel.

The Dutch patent 1 005 190 discloses an apparatus for treating a body cavity or body vessel with a curable material, such as polyurethane (PUR). It is described that the apparatus can be used in the treatment of an aorta aneurysm.

Although considerable attention has been paid to the development of delivery systems for fluid material to a body cavity or vessel, it has been found that the availability of compositions for treating cavities or vessels *in vivo,* is highly unsatisfactory.

In particular, it has been found that known polymer compositions suffer from one or more drawbacks, *e.g.* known compositions have been found to show unsatisfactory handling characteristics, unfavourable curing behaviour, a relatively high level of toxicity, too low biocompatibility, too high thrombogenicity and/or insufficient durability *in vivo.*

It is an object of the present invention to provide a curable polymer composition for use in a treatment that reduces the risk of a future bone fracture, or to reduce the risk of complications of a future bone fracture.

It has been found that this object is achieved by a specific fluid biocompatible polymer composition having a viscosity in a specific range. Accordingly, the present invention relates to a fluid biocompatible polymer composition, suitable for vessel repair - in particular for aneurysm repair-said composition comprising a matrix pre-polymer, a filler and a curing agent, wherein said composition has a viscosity at 25 °C, as measured by a Brookfield viscosimeter, UK, of 2 000-12 000 mm²/sec [cSt] (corresponding to 2-12 Pa.s for a composition with a density of 1 000 kg/m³), which polymer is curable in an aqueous environment at a temperature of 37 °C and wherein said matrix pre-polymer is chosen from the group consisting of silicon (pre)-polymers, polyurethanes and combinations thereof, which biocompatible polymer composition is curable in the presence of a curing catalyst at 37°C to form a cured material with an elongation until rupture of at least 5 % and an elastic modulus of at least 1 MPa.

It has been found that a human or an invertebrate in general may be effectively treated with a curable polymer composition in order to reduce the risk of complications of a future bone fracture.

The bone treated in accordance with this aspect of the invention is preferably a collarbone or a hip.

Typically the (non-broken) bone is provided with a curable polymer composition. Suitable compositions are known in the art, e.g. as described herein or in one of the references cited in the present description.

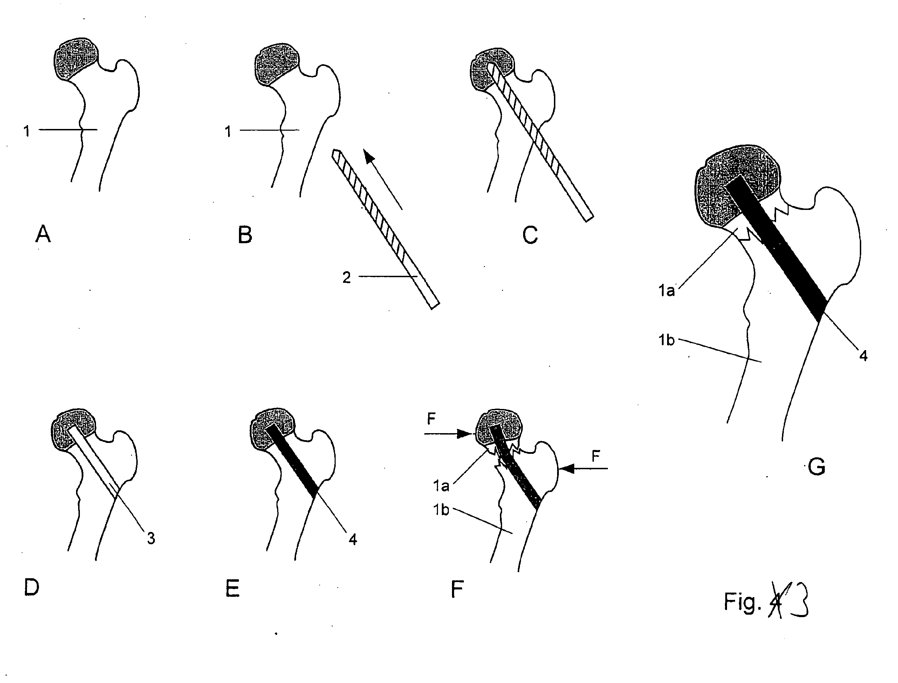

In accordance with a method for treating a bone, a cavity is made in the bone, which may be done by a method generally known in the art, e.g. in a way known to introduce osteosynthetic material into a bone. Thereafter the cavity is provided with the curable composition and the composition is cured. Figure 3 schematically shows by means of an example how a bone may be provided with the curable composition. Figure 3A shows how a drill 2 is directed at a bone 1 and used to drill a hole (figure 3B and 3C). Removal of the drill 2 provides a cavity 3 in the bone (figure 3D) that is filled with the curable composition 5 (figure 3E), which is subsequently allowed to cure. If - at a later moment - the bone is fractured (Figure 3F; arrows F indicate where forces are applied), the cured composition keeps the fractured bone parts 1a and 1b in place, or at least reduces shifting of the bone parts (Figure 3G).

The cavity is preferably provided along at least a substantial part of the bone, for instance by drilling, and filled with the curable composition, after which the composition is cured. The cured composition thus preferably forms an elastic rod-like structure.

If the bone breaks after the composition is cured, the cured composition maintains or swiftly brings back the broken parts essentially in the right position, thus avoiding or at least reducing the risk of complications due to shifting of the bone parts.

Such a method may for instance very suitably be carried out in combination with a surgery that has to be performed on a patient for an acute reason, for instance on a patient of which one of the hips or one of the collarbones has been broken.

Accordingly, the invention also relates to the use of a curable polymer compositions in the manufacture of a physiologically acceptable composition for prophylactic treatment of a bone, preferably a hip or a collarbone. Such prophylactic treatment helps to avoid or at least reduces the risk of complications after fracture.

Moreover, it has been found that satisfactory curing is achieved without excessive heat release.

Such a composition has been found to have particularly favourable handling characteristics. The composition has a suitable flowability to allow introduction of the composition at a site to be repaired in situ through a narrow catheter (*e.g.* with an inner diameter of approximately 1-1.2 mm and a length of about 50-60 cm length) in a sufficiently short time, typically within 1 - 2 min. Further, it has been found that during application, the composition does not leak out of the application system used to introduce the composition *in situ* (*e.g.* at a connection between pump and catheter) if a contrast medium is used for introducing and position the catheter in place. As a contrast medium *e.g.* a water based X-rays contrast fluid, such as Omnipaque® (*e.g.* at a concentration of 300 mg iodine/ml), may be used.

It has been found that a composition according to the invention shows very favourable handling properties before application to a body vessel or cavity. It allows mixing with optionally added other ingredients, *e.g.* as indicated below, adequate application in a fluid state to the site that is to be repaired in an acceptable time-frame to avoid inclusion of anomalies - such as air bubbles - in the composition. Accordingly, a composition according to the invention is preferably essentially free of a gas phase.

It has been found that upon curing of a composition according to the invention, the resulting material has a very good durability in that it is capable of maintaining satisfactory mechanical properties and its integrity for a prolonged time, even up to or exceeding a patient's lifetime.

The viscosity as defined herein is the kinematic viscosity in cSt as measured by Brookfield viscosimeter(UK), model ND J-1 and/or rheometer RMS 800 from Rheometrics USA The kinematic viscosity of a fluid in cSt corresponds to the dynamic viscosity in mPa.s divided by the density of the fluid in g/cm³.

The elongation until rupture is defined herein is the value as measured by a Zwick 1445 tensile strength tester (Germany).

The elastic modulus as defined herein is the value as measured by dynamic mechanical analyser, DMA 7 from Perkin-Elmer (USA).

Preferably, the viscosity of the biocompatible polymer composition is in the range of 3 000 to 10 000 mm²/sec (cSt), more preferably 4 000 to 8 000 mm²/sec (cSt). Particularly good results have been achieved with a composition having a viscosity of approximately 5 000 to 7 000 mm²/sec (cSt). Such a polymer composition is found to have particular satisfactory mechanical properties (after curing), in combination with sufficient flowability (before curing).

After curing, the elongation until rupture of the cured composition is preferably at least 10 %, more preferably at least 25 % even more preferably at least 50 %. The upper limit is in practice not particularly important. Very good results have for example been reached with a cured composition having an elongation until rupture of 500 % or less, more in particular of 250 % or less.

The elastic modulus is preferably at least 2 MPa, more preferably at least 3 MPa, even more preferably at least 4 MPa.

The upper limit is not particularly critical. Very good results have been achieved with a composition having an elastic modulus of less than 20 MPa, after curing.

It has been found that a composition giving rise to a elastic modulus of about 5-15 MPa, is particularly advantageous with respect to the durability of the cured material under *in vivo* aortic conditions.

Preferably, after curing of the composition the resulting material has a stress value of at least 5 kPa at 1 % strain, more preferably of at least 30 kPa at 20% strain, even more preferably a stress value of at least 1 MPa at 50 % strain (As determined with Zwick 1445 or with DMA 7, Perkin-Elmer).

The glass transition temperature (Tg) of a cured material obtained from a composition according to the invention is typically less than 37 °C. Preferably the Tg is less than 25 °C. Very good results have been achieved with a material having a Tg of less than -25 °C. (Tg is the value as measured by differential scanning calorimetry (DSC) on a DSC 7, Perkin-Elmer.

The composition is preferably chosen such that the absolute value of the curing enthalpy of the biocompatible polymer composition is less than 10 J/g, as measured by determining the heat production during curing by DSC (DSC 7, Perkin-Elmer).

The components of a composition according to the invention can be prepared by mixing the component in a common mixing device. Particularly suitable is a high shear mixer or an ultrasonic mixer. Suitable mixing conditions can be routinely determined by the skilled person based upon common general knowledge and the information disclosed herein. Very suitable is for example an intermittent mixing procedure (*e.g.* at 20 000 to 30 000 rpm for a 100 ml composition), wherein between periods of mixing (e.g. of 1-20 min) the composition is allowed to rest in order to cool down (*e.g.* for 1 to 10 min).

The components or the composition itself should preferably be sterile or sterilisable. At least when present in the composition, the components should have a sufficient hemocompatibility, when used in a blood vessel and are preferably non-thrombogenic and non-tumorogenic.

The term matrix pre-polymer is used herein to describe a curable monomer, a curable oligomer or a curable polymer. Before curing the matrix pre-polymer typically comprises one or more functional groups that allow further polymerisation, *e.g.* by cross-linking, to form the matrix of the composition after it has been cured. It is stressed that the terms pre-polymer and polymer are to be interpreted broadly, with respect to the number of monomeric units.

The number of monomeric units or molecular weight of the matrix pre-polymer is not particularly critical, as long as it provides a suitable viscosity in the composition. Good results have been obtained with a matrix pre-polymer having at least 1, preferably at least 5, more preferably at least 20 monomeric units, before curing is initiated. For practical reasons, the matrix-polymer generally comprises less than 20 000 monomeric units, preferably less than 1 000, more preferably less than 100, before curing is initiated.

Very good results have been achieved with a composition comprising a matrix pre-polymer having 1 to 5 monomeric units.

The number average molecular weight of the matrix pre-polymer may for example be in the range of 500 to 400 000 gram/mol, preferably in the range of 6 000 to 280 000 gram/mol. As a matrix pre-polymer, any biocompatible pre-polymer can be used that is curable under physiological conditions into a material with satisfactory mechanical properties and that can be delivered to the site inside the body where it is to cure.

The amount of matrix prepolymer can be chosen within wide limits, depending upon the desired viscosity and other properties and may be adequately determined by the skilled professional. Preferably the concentration of the matrix pre-polymer is 10 to 85 wt. % based on the total weight of the composition, more preferably the concentration is in the range of 50 to 75 wt. %. Very good results have been achieved with a composition having an amount of matrix pre-polymer in the range of 60 to 70 wt. %. Examples of suitable matrix pre-polymers are silicon (pre-)polymers, polyurethanes, and combinations thereof such as blends, hybrid polymers and copolymers comprising silicon (pre-)polymers and/or polyurethanes.

Particular suitable hybrid polymers are polymers filled with a molecular silica. A particular preferred molecular silica in a hybrid polymer is a polyhedral oligomeric silsesquioxane (P.O.S.S.). Such a molecular silica is commercially available from Hybrid Plastics, USA under the trademark POSS^{TM}.

Preferably the pre-polymer reacts essentially without forming a rest-product, such as a gas (e.g CO₂) or another residual product. Such rest products may be detrimental to the properties, in particular physical properties, of the cured composition.

A preferred matrix pre-polymer is a silicon (pre-)polymer, which is an example of a pre-polymer that usually reacts without forming a rest product. It has been found that a composition comprising a silicon (pre-)polymer is not only very suitable for repairing an aneurysm in such a way that the repaired blood vessel has very satisfactory mechanical properties for a prolonged period of time, but that such a (pre-)polymer in a composition according to the invention also offers the advantages of being hemocompatible, non-thrombogenic, highly biocompatible and non-carcinogenic.

Further it has been found that a composition comprising a silicon (pre-)polymer is capable of curing fast, typically within 5 min. or less-preferably within 2-3 min or less - without generating excessive heat that may do damage to the body or may give rise to defects in the cured material.

The durability of cured silicon (pre-) polymer material has been found to be very high. Life-times of 10-40 years or more are considered to be feasible (based upon stretch test in an in vitro simulation, simulating heart function at 60 beats per minute).

Preferably a silicon (pre-)polymer used in a composition according to the invention has a start viscosity (i.e. before mixing it to a composition according to the invention) of at least 300 mm²/sec (cSt). More preferably the start viscosity is in the range of 300 to 1 500 mm²/sec (cSt).

Highly preferred is a polydialkylsiloxane polymer, comprising at least two vinyl groups, preferably at the terminal ends. Very good results have been achieved with a polydialkylsiloxane polymer having 3-5 vinyl groups.

Very good results have been achieved with a polydimethylsiloxane monopolymer or a polydialkylsiloxane copolymer - e.g. a block copolymer, a random copolymer or an alternating copolymer - mainly comprising dimethylsiloxane units. A highly preferred polydimethylsiloxane polymer is shown in formula 1

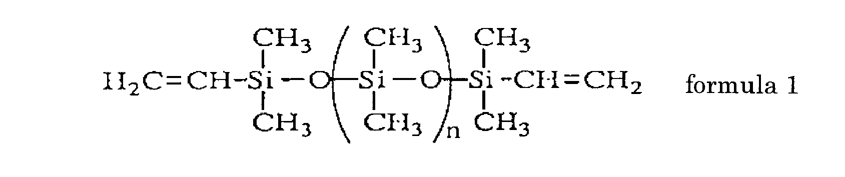

Preferably the number average weight in case this polymer is used is chosen in the range of 20 000 to 200 000 g/mol.

A silicon (pre-)polymer is in practice preferred over polyurethanes. It has been found that in a composition wherein the matrix pre-polymer is a polyurethane pre-polymer, the heat generation tends to be quite large. Further the CO₂ formed during the polymerisation may adversely affect physical properties such as the elongation up to rupture, the elastic modulus and the durability.

Curing can be achieved in many ways, *e.g.* by the presence of a curing agent and/or by irradiation, in particular irradiation with UV light. The use ofUV-light may for example be suitable in case of a local repair. In practice, generally no exposure to radiation is required.

Preferably curing occurs by the presence of a curing agent. The use of a curing agent is particularly preferred. A curing agent as defined herein is any agent that can react with the matrix pre-polymer to result in a solidification (*e.g.* by cross-linking or gelling) of the composition. Curing agents include cross-linking agents and agents that cure the composition in any other way. Preferably the curing agent is a cross-linking agent. The curing agents can be chosen from the group of curing agents that are suitable to react with the chosen matrix pre-polymer.

A suitable amount of curing agent can easily be determined, depending upon the type of curing agent and the quantity and nature of the other components in the composition. Preferably, the curing agent is present in an amount of at least 0.1 wt. % based on the total weight of the composition, more preferably at least 5 wt. %. The amount of curing agent is preferably less than 15 wt. %, more preferably less than 10 wt. %.

Preferably the curing agent is present in the composition in an amount providing a number of functional groups in the range of 1-10 times the number of functional groups that is provided by the matrix pre-polymer.

Functional groups, as used herein, are those functional groups that are capable of participating in the curing, in particular by being capable of reacting with a functional group of another molecule (curing agent or matrix pre-polymer) in the composition. Examples of functional groups are vinyl groups, acryloyl groups, methacryloyl groups and hydride groups. Vinyl groups are in particular preferred in the matrix pre-polymer. Hydride groups are in particular preferred in the curing agent.

Examples of suitable curing agents are polyalkylhydrosiloxane polymers, including fluorinated polyalkylhydrosiloxane polymers, functionalised molecular silica compounds, such as Vinyl Q® and P.O.S.S. compounds. Very good results, in particular in combination with a silicon (pre-)polymer as a matrix pre-polymer, have been achieved with a polyalkylhydrosiloxane polymer. When used in combination with a silicon (pre-)polymer as a matrix pre-polymer, the molar ratio of hydride to vinyl functional groups is preferably 1:1 to 10:1.

A preferred polyalkylhydrosiloxane polymer as a curing agent is a copolymer of alkylhydrosiloxane moieties and dialkylsiloxane moieties, preferably of methylhydrosiloxane moieties and dimethylsiloxane moieties.

Preferably the amount of dialkylsiloxane moieties - in particular dimethylsiloxane moieties - and/or the amount of alkylhydrosiloxane moieties-in particular methylhydrosiloxane moieties - in a polyalkylhydrosiloxane polymer is 1-100, and more preferably 5 to 20. The dialkylsiloxane-alkylhydrosiloxane copolymer may be a random, alternating or block copolymer.

As a filler any physiologically acceptable filler can be used. The filler may for example by essentially spherical, filament-like, disc-like or an agglomerate of smaller nanofiller particles.

In practice, the number average particle diameter of the fillers is in the range of 10 to 50 000 nm, preferably in the range of 10-1 000 nm, more preferably in the range of 10 to 500 nm

Very good results have been achieved with a nano-particle filler which have a relatively high specific area ( characterised by a high BET-value), typically in the range of 50 to 400 m²/g., in particular with hybrid-nanofillers *i.e.* a filler that not only acts as a filler, but that also contains reactive sites, *e.g.* vinyl groups, that are capable of participating in the curing of the composition. A hybrid-nanofiller has not only been found to have a positive effect on the mechanical properties after curing, but is also found that these hybrid fillers do not lead to adverse physiological reactions, or at least are less prone to lead to adverse physiological reactions in comparison to several ordinary fillers.

It is also possible to use a combination of different fillers. It has for example been found that a combination of a hybrid-nanofiller, in particular a silica based hybrid-nanofiller, in combination with a hydrophobic filler , in particular a silica hydrophobic filler.

The amount of filler in the composition depends inter alia on the type of filler, its contribution to the viscosity of the composition. Also the desired characteristics of the composition after curing may play a role in determining the concentration. The skilled professional will readily be able to determine a suitable concentration. Typically the filler is present in an amount of at least 1wt. %, based on the total weight. The upper limit is essentially determined by the amount of other constituents, present in the composition. For practical reasons, the amount of filler is usually less than 50 wt. %, based upon the total weight of the composition. Preferably the concentration is at least 2 wt. %, more preferably at least 15 wt. %. The amount of filler is preferably less than about 45 wt. %, more preferably less than about 40 wt.%. Very good results have been achieved with a filler concentration of 25 to 45 wt. %.

Examples of suitable fillers are fillers selected from the group consisting of silica, clay, mica, calcium carbonate, fibre glass, polyethylenenaphthalate and combinations thereof, which may be present in unmodified or chemically modified form. Preferred is a nano-sized silica filler, *e.g.* a molecular silica filler. It has been found that silica not only contributes particular well to mechanical properties, but also is suitable as a contrast agent, e.g. for X-ray monitoring.

Preferably, the surface of the filler is hydrophobic, *e.g.* due to chemical modification. The term hydrophobic filler is used herein to describe a filler, of which the surface has been treated with a non-polar compound that dissolves better (*i.e.* has a higher solubility) in an organic solvent such as an alkane than in water.

It has been found that such a filler contributes very satisfactorily to the mechanical properties of the composition after curing and also has only a relatively small effect on the flowability in comparison to hydrophilic fillers.

A preferred hydrophobic filler is a filler, more preferably a silica filler, wherein at least the surface has been modified with an organosilicon compound, e.g. with dichlorodimethylsilane ((CH₃)₂SiCl₂), hexamethyldisilazane ([(CH₃)₃Si]₂NH) or reactive polydimethylsiloxanes. Such fillers are readily available in the market, e.g. Aerosil R8200 (Degussa), functionalised molucular silica (*e.g.* Vinyl Q® (Gelest, USA)). Besides the excellent mechanical properties of a cured material, obtained from a composition comprising a surface modified silica filler, it has been found that such a filler may also serve as a contrast agent as an alternative to commonly used separate contrast agents, or to be used in combination with (reduced concentration of) such a contrast agent.

Very good results have been realised with a filler modified with vinylalkylsiloxane, in particular a filler modified with vinyldimethylsiloxane. A cured material based upon a composition comprising vinylalkylsiloxane modified filler has been found to have particularly good mechanical properties and durability, after implementation in the body, in particular in combination with a silicon (pre-)polymer as a matrix pre-polymer and/or a alkylhydrosiloxane as a curing agent. Besides good mechanical properties for a prolonged period, it has been found that such a composition has a very advantageous light transparency and curing behaviour.

A composition according to the invention may essentially consist of matrix pre-polymer, curing agent and filler. A composition may comprise one or more additives, e.g. one or more additives selected from the group consisting of contrast agents, curing inhibitors and chain extenders.

As indicated above, in particular silica may be present not only as a filler but also as a contrasting agent. Other examples of particularly suitable contrast agents include iodine based contrast agents (containing iohexol e.g. Omnipaque®,(supplied by Nycomed). An iodine based contrast agent (such as Omnipaque ®) is a preferred contrast agent, in that it has been found that it does not adversely affect the curing process.
A chain extender may be present in a composition to change the elasticity modulus and/or the elongation until rupture of a cured material with. Suitable examples of chain extenders include hydride terminated polydimethyl siloxanes.

Very good results have been obtained with a chain extender, present in a composition according to the invention in a concentration of 1-10 wt. % based upon the combined weight of filler, matrix pre-polymer and curing agent..

Curing inhibitors may be present in the composition to improve stability of the composition until the curing is induced, shortly before application of the composition. An examples of a curing inhibitors is 1,3,5,7-tetravinyl-1, 3, 5, 7-tetramethylcyclotetrasiloxane .

The curing may be initiated and propagated in any way suitable to cure the particular combination of matrix pre-polymer and curing agent as long as this curing is physiological acceptable. The curing may for example be started under influence of radiation, *e.g.* electromagnetic radiation or chemically, *e.g.* by an added catalyst or reactant or by a component that is naturally present in the body, *e.g.* water or particular ions..

Preferably the curing is performed under influence of a curing catalyst. The curing catalyst may for example be present in a composition comprising a matrix pre-polymer, a filler and/or other additives. In particular if such a curing catalyst composition comprises a matrix pre-polymer, it will usually be essentially free of curing agent.

It has been found that by using a curing catalyst, a cured material is obtained that has very favourable mechanical properties throughout the material.

Very good results have been achieved with a platinum catalyst, in particular for curing a composition comprising a matrix pre-polymer with vinyl units as reactive site and a curing agent with hydride units as active site.

Highly preferred examples of platinum catalysts are platinum complexes, in particular platinum complexes selected from the group consisting of platinum-divinyltetramethyldisiloxane complexes. Good results have *inter alia* been realised when using such a complex in a concentration of 2 to 7 ppm Pt based upon the total weight of the composition.

The concentration of curing catalyst can readily be determined depending upon the composition and the desired curing time. For example for curing a composition comprising a silicon (pre-)polymer, in particular a polydialkylsiloxane polymer comprising at least two vinyl groups, and a polyalkylhydrosiloxane polymer, good results have been achieved with a platinum catalyst in a concentration of at least 5 ppm (based upon the total weight of the biocompatible polymer composition). Particular favourable with respect to the curing time has been found to be a concentration of about 5 to 500 ppm.

The curing catalyst is preferably mixed with the composition, shortly before applying the composition to the body vessel or cavity. Very good results have been achieved by mixing a composition comprising a curing catalyst with a biocompatible polymer composition according to the invention in a static mixer, in particular in a static mixer comprising at least 30 mixing elements. Static mixers are readily available in the market. They comprise an elongated channel wherein for example a multi-helical screw is placed along which the fluids are passed and mixed. Herein each winding of the screw is considered as a single mixing element.

The polymer composition can be introduced at the site that is to be treated by any delivering system suitable for delivering fluid compositions in a body.

The invention is further illustrated by the following examples

### Example 1

Polydimethyldiloxane (PDMS) vinyl terminated oligomers (supplied by Gelest, USA) were mixed with various weight percent of filler (Vinyl Q®) (supplied by Gelest, USA) in the range 0 to 50 % w/w and with different hydride methyl siloxane (hydride HMS-301^{TM}, supplied by ABCR, Germany. concentrations as well as different platinum concentrations, The platinum compound was platinum-divinyltetramethyldisiloxane complex, 3-3.5% platinum concentration in vinylterminated ploydimethylsiloxane, supplied by ABCR, Germany.

The formulations providing the best viscosity and mechanical properties following the protocol are obtained at a weight percent above 25% of vinyl Q® in 1000 cSt PDMS vinyl terminated. The viscosity of the formulation containing 20% w/w Vinyl Q® in 1000 cSt PDMS vinyl terminated oligomer was found to be 4 000 cSt.

The addition of 5% Aerosil R8200^{TM} (supplied by Degussa, Germany) to the 20% w/w Vinyl Q®, was found to accelerate the cure rate, increase the mechanical properties. The viscosity increase varied from no significantly increase in the start viscosity up to an acceptable increase from 4 000 to 5 000 cSt.

The 50% w/w Vinyl Q® in 1 000 cSt PDMS vinyl terminated provided a viscosity of 7 000 cSt.

### Example 2

The following basic formulation was made:
- 20% w/w vinyl Q® (molecular filler)
- 70%w/w 1000 cSt PDMS vinyl terminated
- 10% w/w silica Aerosil R8200^{TM}

The composition was prepared as follows: The vinyl Q® was added to the PDMS. After gently mixing until a homogenous mixture was formed the silica Aerosil R8200^{TM} was added in small quantities at a time to 200 g of PDMS/Vinyl Q®, and was vigorously mixed with a Kinematica high shear mixer (Kinematica PT-MR 3000 EU/502) for one hour at 25 000 rpm. The resulting mixture was homogenous.

From the resulting basic formulation of PDMS/vinyl Q®/silica two components (A and B) were prepared. Component A contained 6 g hydride having 10 functional (HMS-301) in 24 g of PDMS/vinyl Q®/silica, and component B contained 2.4 g platinum from a 0.0286% complex platinum (as in Example 1) added to 26 g PDMS/vinyl Q®/silica. The material was cured at to form sheets of 10 cm x 10 cm x 2 mm. It was found that the chain extender influenced the cured material, by reducing the tensile modulus and increasing the maximum elongation.

In a tensile strain test, a sample of the cured material was strained at 60% from its original length and subjected to 100% stretch at 1260 cycles per minute. The sample was tested for a life time equivalent to at least 10 years of stretching in *vivo* (heart rate 60 BPM). The properties still were satisfactory at the end of the test.

### Example 3

The following basic formulation was made as indicated in Example 2
- 30 % Vinyl Q® w/w
- 67 % 1000 cSt PDMS vinyl terminated
- 3 % w/w silica Aerosil R8200^{™}

The component A contained 7 g HMS-301^{™} (9% of the total formulation weight), plus 23 g of the basic 30% w/w Vinyl Q® formulation given above, while component B contained 3 g platinum at 0.0286% complex platinum in PDMS 1000 cSt plus 27 g from the basic formulation (with 30% Vinyl Q®).

A cylindrical test sample was produced by mixing component A & B and curing the composition. Curing was performed at room temperature to produce sheets as indicated in Example 2. In addition, a cylindrical aneurysm-type of shape was produced to fit the material to be tested in long term experiments. The cylindrical test sample was connected to an air pressure at 0.5 bar, inflating each 0.3 seconds for two months before stopping the experiment. The sample did not show any signs of fatigue or deterioration.

### Example 4

Four samples (with a total weight of 60 grams) were produced based on 1000 cSt PDMS vinyl terminated, each having the same filler concentration of 35.5 wt. % of Vinyl Q®, 5 wt.% silica from Aerosil R8200^{™}, each having the same platinum concentration 0.2 g at 3 wt. % complex platinum in PDMS 1 000 cSt., but having different hydride concentration (HMS-301^{™}).

The HMS-301^{™} quantities per 60 g sample were as follows:
Example 4.1: 6.0 gr. HMS-301^{™}
Example 4.2: 6.5 gr. HMS-301^{™}
Example 4.3: 7.0 gr. HMS-301^{™}
Example 4.5: 7.5 gr. HMS-301^{™}

Cured samples (as indicated in Example 3) were connected to an air pressure of 0.5 bar, inflating each 0.3 seconds. After 4 months of testing, the sample did not show any signs of fatigue or deterioration.

Example 4.2 showed the best results with respect to Young' modulus, elongation and tear resistance.

### Example 5

The following basic formulation was made
- 49 wt. % Vinyl Q®
- 1.1 wt. % silica from Aerosil R8200^{™}
- 49.9 wt % 1000 cSt PDMS vinyl terminated

As component A, a mixture was made containing 11 g HMS and 19 g from the 49% w/w vinyl Q® basic formulation, and component B containing 0.4 gr platinum at 3% complex platinum in PDMS 1000 cSt and 29.6 gr from the same basic Vinyl Q® formulation.

It was found that a cured test sample produced with this composition was more rigid than any of the samples made in Examples 1-4 or a test sample made with clay or mica as nanofiller.

A cylindrical test sample was connected to an air pressure of 0.5 bar, inflating each 0.3 seconds. After 4 months of testing, the sample did not show any signs of fatigue or deterioration.

### Example 6

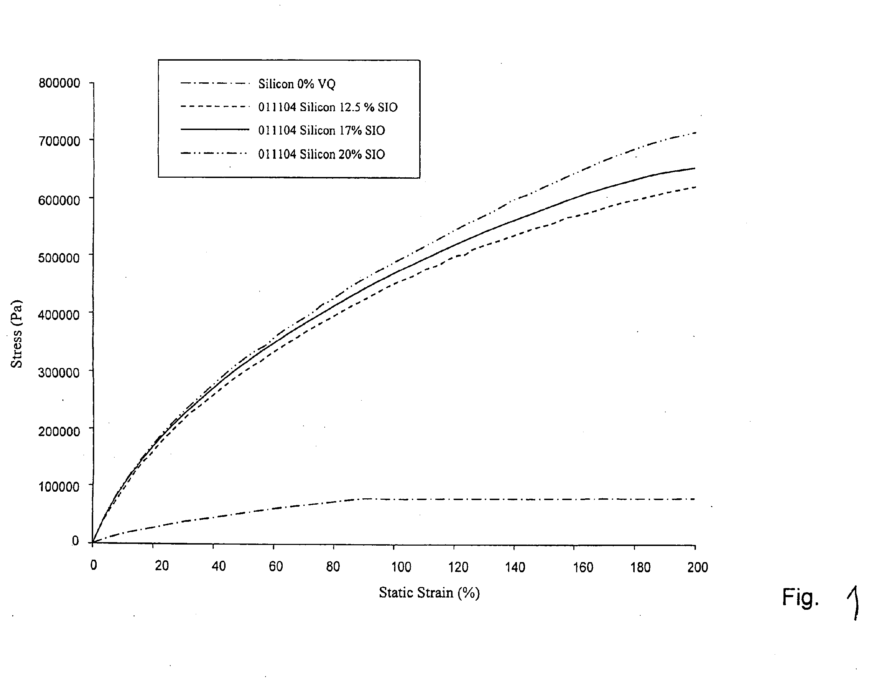

The modulus of cured silicone compositions, were measured using the Dynamic Mechanical Analyser (DMA 7 from Perkin Elmer), in the stress-strain mode. The strength until sample rupture was determined from samples which were hold between air-pressurised grips within the Zwick 1445. The mechanical properties were observed to be reproducible within an error of 5%. The effect of the percent of added silica filler on the overall mechanical properties such as stress and strain are shown in Figure 1. The elastic modulus of a cured composition is determined by the initial slope of the corresponding curve.

There was a huge stress difference between a silicone system containing 0% silica and that containing 12.5%. The static strain was not significantly different between the different samples until 200% stretching.

Also, no appreciable difference in stress existed in between silicone systems having 12.5 % and those having 20% silica.

The silicone/silica systems having 20% silica showed the highest tear strength.

### Example 7.

The stress strain curve of the following systems was determined: silicone 0% silica, silicone with 20% vinyl Q® and 7% silica, silicone with 30% Vinyl Q®, silicone with 34% vinylQ®, silicone 34% Vinyl Q® and 4% silica with 1% microfibres, silicone with 34 % vinyl Q® and 4% silica, and silicone with 35% vinyl Q® and 5% silica. Otherwise the conditions were as indicated in the previous Examples.

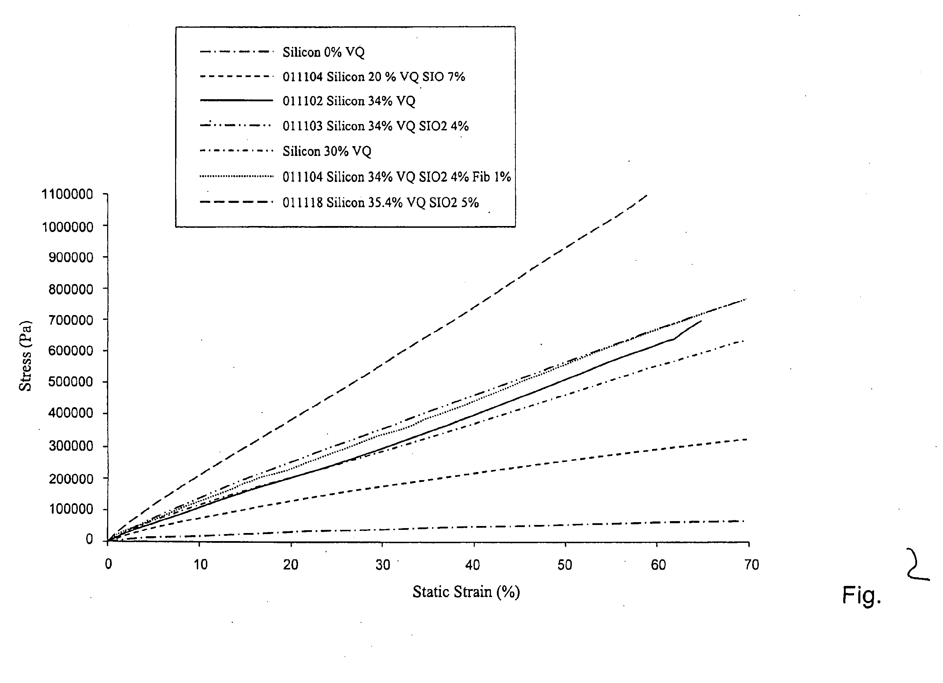

The results are shown in Figure 2.

### Example 8

The Vinyl Q®/PDMS/silica nanofiller formulations of examples 1-7 were put in glass pots and were brought to 200°C for 15 minutes. No effect of temperature on the pre-polymerisation and/or crosslinks was noticed, which demonstrates that a silicon (pre-)polymer based composition according to the invention can easily be sterilized.

## Claims

1. A biocompatible polymer composition, suitable for *in vivo* vessel repair, comprising a matrix pre-polymer, a filler and a curing agent, wherein said composition has a viscosity of 2 000 to 12 000 mm²/sec [cSt] at 25 °C wherein said biocompatible polymer composition is curable in the presence of a curing catalyst at 37 °C to form a cured material with an elongation until rupture of at least 5 % and an elastic modulus of at least 1 MPa, and wherein said matrix pre-polymer is chosen from the group consisting of silicon (pre)-polymers, polyurethanes and combinations thereof, for prophylactic treatment of a bone.

2. Composition according to claim 1, wherein the viscosity of the biocompatible polymer composition is in the range of 3 000 to 10 000 mm²/sec [cSt], preferably of 4 000 to 8 000 mm²/sec [cSt].

3. Composition according to claim 1 or 2, wherein said biocompatible polymer composition is curable in the presence of a curing catalyst at 37 °C to form a cured material with an elongation until rupture of at least 10 %, preferably at least 25 %.

4. Composition according to any of the preceding claims, wherein the matrix pre-polymer is a silicon (pre-)polymer, preferably a polydialkylsiloxane (pre-)polymer comprising at least two vinyl groups more preferably, more preferably polydialkylsiloxane (pre-)polymer comprising three to five vinyl groups.

5. Composition according to any of the preceding claims, wherein the filler is a hydrophobic filler.

6. Composition according claim 5, wherein the hydrophobic filler is modified with an organosilicon compound, preferably with a vinylalkylsiloxane.

7. Composition according to any of the preceding claims, wherein the biocompatible polymer composition comprises a curing-inhibitor.

8. Composition according to any of the preceding claims, wherein the concentration of the matrix pre-polymer is 10 to 85 wt. % based on the total weight of the composition, preferably 50-70 wt. %.

9. Composition according to any of the preceding claims, wherein the curing agent is present in an amount of at least 0.1 wt. % based on the total weight of the composition.

10. Composition according to any of the preceding claims, wherein the curing agent is a polyalkylhydrosiloxane polymer, preferably a polyalkylhydrosiloxane copolymer comprising alkylhydrosiloxane moieties and dialkylsiloxane moieties, more preferably comprising methylhydrosiloxane moieties and dimethylsiloxane moieties.

11. Composition according to any of the preceding claims, wherein the curing agent is present in an amount providing a number of functional groups in the range of 1-10 times the number of functional groups that is provided by the matrix pre-polymer.

12. Composition according to any of the preceding claims, wherein the filler is present in an amount of 1-50 wt. %, preferably 2-45 wt. %, more preferably 15-40 wt. %, based on the total weight of the composition.

13. Composition according to any of the preceding claims, wherein the composition comprises at least one filler selected from the group consisting of silica nanofillers, molecular silica, clay nanofillers, mica nanofillers, polymeric microfibres and glass microfibres.

14. Composition according to any of the preceding claims, comprising a chain extender.

15. Composition according to any of the preceding claims, wherein the number average particle size of the filler is chosen in the range of 10 to 50 000 nm, preferably 10 to 1 000 nm, more preferably 10 to 500 nm.

16. Kit of parts suitable for use in an *in vivo* vessel repair, comprising a biocompatible polymer composition according to any of the claims 1-15, and a curing-catalyst composition, for prophylactic treatment of a bone.

17. Kit according to claim 16, wherein the curing catalyst composition comprises at least one component selected from the group consisting of matrix pre-polymers, fillers and contrast agents.

18. Kit according to claim 16 or 17, wherein the viscosity of the curing catalyst composition is at most 1 500 mm²/sec [cSt] higher or lower than the viscosity of the biocompatible polymer composition.

19. Kit according to any of the claims 16-18, wherein the biocompatible polymer composition mixed with the curing catalyst composition, has a curing time of 5 min or less, preferably of less than 3 min.

20. Composition according to any of the claims 1-15, wherein the bone is a hip or a collarbone.

## Patentansprüche

1. Biokompatible Polymerzusammensetzung, die zur In-vivo-Gefäßreparatur geeignet ist, umfassend ein Matrix-Präpolymer, einen Füllstoff und ein Härtungsmittel, wobei die Zusammensetzung eine Viskosität von 2.000 bis 12.000 mm²/sec [cSt] bei 25°C aufweist, wobei die biokompatible Polymerzusammensetzung in Gegenwart eines Härtungskatalysators bei 37°C unter Bildung eines gehärteten Materials mit einer Dehnung bis zum Bruch von mindestens 5% und einem Elastizitätsmodul von mindestens 1 MPa härtbar ist, und wobei das Matrix-Präpolymer ausgewählt ist aus der Gruppe, bestehend aus Silicium-(Prä)Polymeren, Polyurethanen und Kombinationen davon, zur prophylaktischen Behandlung eines Knochen.

2. Zusammensetzung gemäß Anspruch 1, wobei die Viskosität der biokompatiblen Polymerzusammensetzung im Bereich von 3.000 bis 10.000 mm²/sec [cSt], vorzugsweise von 4.000 bis 8.000 mm²/sec [cSt], liegt.

3. Zusammensetzung gemäß Anspruch 1 oder 2, wobei die biokompatible Polymerzusammensetzung in Gegenwart eines Härtungskatalysators bei 37°C unter Bildung eines gehärteten Materials mit einer Dehnung bis zum Bruch von mindestens 10%, vorzugsweise mindestens 25%, härtbar ist.

4. Zusammensetzung gemäß einem der vorangehenden Ansprüche, wobei das Matrix-Präpolymer ein Silicium-(Prä)Polymer, vorzugsweise ein Polydialkylsiloxan-(Prä)Polymer, umfassend mindestens zwei Vinylgruppen, stärker bevorzugt ein Polydialkyl-siloxan-(Prä)Polymer, umfassend drei bis fünf Vinylgruppen, ist.

5. Zusammensetzung gemäß einem der vorhergehenden Ansprüche, wobei der Füllstoff ein hydrophober Füllstoff ist.

6. Zusammensetzung gemäß Anspruch 5, wobei der hydrophobe Füllstoff mit einer Organosilicumverbindung, vorzugsweise mit einem Vinylalkylsiloxan, modifiziert ist.

7. Zusammensetzung gemäß einem der vorhergehenden Ansprüche, wobei die biokompatible Polymerzusammensetzung einen Härtungsinhibitor umfasst.

8. Zusammensetzung gemäß einem der vorhergehenden Ansprüche, wobei die Konzentration des Matrix-Präpolymers 10 bis 85 Gew.-%, bezogen auf das Gesamtgewicht der Zusammensetzung, vorzugsweise 50 bis 70 Gew.-%, beträgt.

9. Zusammensetzung gemäß einem der vorhergehenden Ansprüche, wobei das Härtungsmittel in einer Menge von mindestens 0,1 Gew.-%, bezogen auf das Gesamtgewicht der Zusammensetzung, vorhanden ist.

10. Zusammensetzung gemäß einem der vorhergehenden Ansprüche, wobei das Härtungsmittel ein Polyalkylhydrosiloxanpolymer, vorzugsweise ein Polyalkylhydrosiloxancopolymer, umfassend Alkylhydrosiloxangruppierungen und Dialkylsiloxangruppierungen, stärker bevorzugt umfassend Methylhydrosiloxangruppierungen und Dimethylsiloxangruppierungen, ist.

11. Zusammensetzung gemäß einem der vorhergehenden Ansprüche, wobei das Härtungsmittel in einer Menge vorhanden ist, die eine Anzahl funktioneller Gruppen im Bereich des 1-10-Fachen der Anzahl funktioneller Gruppen, die durch das Matrix-Präpolymer bereitgestellt wird, bereitstellt.

12. Zusammensetzung gemäß einem der vorhergehenden Ansprüche, wobei der Füllstoff in einer Menge von 1-50 Gew.-%, vorzugsweise 2-45 Gew.-%, stärker bevorzugt 15-40 Gew.-%, bezogen auf das Gesamtgewicht der Zusammensetzung, vorhanden ist.

13. Zusammensetzung gemäß einem der vorhergehenden Ansprüche, wobei die Zusammensetzung mindestens einen Füllstoff, ausgewählt aus der Gruppe, bestehend aus Siliciumdioxid-Nanofüllstoffen, molekularem Siliciumdioxid, Ton-Nanofüllstoffen, Glimmer-Nanofüllstoffen, Polymermikrofasern und Glasmikrofasern, umfasst.

14. Zusammensetzung gemäß einem der vorhergehenden Ansprüche, umfassend ein Kettenverlängerungsmittel.

15. Zusammensetzung gemäß einem der vorhergehenden Ansprüche, wobei die zahlenmittlere Partikelgröße des Füllstoffs in dem Bereich von 10 bis 50.000 nm, vorzugsweise 10 bis 1.000 nm, stärker bevorzugt 10 bis 500 nm, gewählt wird.

16. Kit, der zur Verwendung in einer In-vivo-Gefäßreparatur geeignet ist, umfassend eine biokompatible Polymerzusammensetzung gemäß einem der Ansprüche 1-15 und eine Härtungskatalysatorzusammensetzung, zur prophylaktischen Behandlung eines Knochens.

17. Kit gemäß Anspruch 16, wobei die Härtungskatalysatorzusammensetzung mindestens eine Komponente, ausgewählt aus der Gruppe, bestehend aus Matrix-Präpolymeren, Füllstoffen und Kontrastmitteln, umfasst.

18. Kit gemäß Anspruch 16 oder 17, wobei die Viskosität der Härtungskatalysatorzusammensetzung höchstens 1.500 mm²/sec [cSt] höher oder niedriger ist als die Viskosität der biokompatiblen Polymerzusammensetzung.

19. Kit gemäß einem der Ansprüche 16 bis 18, wobei die biokompatible Polymerzusammensetzung, die mit der Härtungskatalysatorzusammensetzung gemischt wird, eine Härtungszeit von 5 min oder weniger, vorzugsweise von weniger als 3 min, aufweist.

20. Zusammensetzung gemäß einem der Ansprüche 1 bis 15, wobei der Knochen eine Hüfte oder ein Schlüsselbein ist.

## Revendications

1. Composition polymère biocompatible adaptée pour la réparation vasculaire *in vivo*, comprenant un prépolymère matriciel, une charge et un agent durcisseur, **caractérisée en ce que** ladite composition présente une viscosité comprise entre 2 000 et 12 000 mm²/s [cSt] à 25 °C, **en ce que** ladite composition polymère biocompatible peut être durcie en présence d'un catalyseur de durcissement à 37 °C pour former un matériau durci présentant un allongement à la rupture d'au moins 5% et un module d'élasticité d'au moins 1 MPa, et **en ce que** ledit prépolymère matriciel est choisi dans le groupe composé des (pré)polymères de silicium, des polyuréthanes et des combinaisons de ceux-ci, en vue d'un traitement prophylactique d'un os.

2. Composition selon la revendication 1, **caractérisée en ce que** la viscosité de la composition polymère biocompatible est comprise entre 3 000 et 10 000 mm²/s [cSt], de préférence entre 4 000 et 8 000 mm²/s [cSt].

3. Composition selon la revendication 1 ou la revendication 2, **caractérisée en ce que** ladite composition polymère biocompatible peut être durcie en présence d'un catalyseur de durcissement à 37 °C pour former un matériau durci présentant un allongement à la rupture supérieur ou égal à 10 %, et de préférence supérieur ou égal à 25 %.

4. Composition selon l'une quelconque des revendications précédentes, **caractérisée en ce que** le prépolymère matriciel consiste en un (pré)polymère de silicium, de préférence un (pré)polymère de polydialkylsiloxane comprenant au moins deux groupes vinyl, et de manière plus préférentielle un (pré)polymère de polydialkylsiloxane comprenant trois à cinq groupes vinyl.

5. Composition selon l'une quelconque des revendications précédentes, **caractérisée en ce que** la charge est une charge hydrophobe.

6. Composition selon la revendication 5, **caractérisée en ce que** la charge hydrophobe est modifiée par un composé organosilicique, de préférence un vinyl alkyl siloxane.

7. Composition selon l'une quelconque des revendications précédentes, **caractérisée en ce que** la composition polymère biocompatible comprend un inhibiteur de durcissement.

8. Composition selon l'une quelconque des revendications précédentes, **caractérisée en ce que** la concentration de prépolymère matriciel est comprise entre 10 et 85 %, de préférence 50 à 70 % du poids total de la composition.

9. Composition selon l'une quelconque des revendications précédentes, **caractérisée en ce que** l'agent durcisseur est présent à hauteur d'au moins 0,1 % du poids total de la composition.

10. Composition selon l'une quelconque des revendications précédentes, **caractérisée en ce que** l'agent durcisseur est un polymère de polyalkylhydrosiloxane, de préférence un copolymère de polyalkylhydrosiloxane comprenant des fractions alkylhydrosiloxane et des fractions dialkylsiloxane, comprenant de façon plus préférentielle des fractions méthylhydrosiloxane et des fractions diméthylsiloxane.

11. Composition selon l'une quelconque des revendications précédentes, **caractérisée en ce que** la quantité présente d'agent durcisseur est propre à fournir un nombre de groupements fonctionnels de 1 à 10 fois le nombre de groupements fonctionnels fournis par le prépolymère matriciel.

12. Composition selon l'une quelconque des revendications précédentes, **caractérisée en ce que** la charge est présente à hauteur de 1 à 50 %, de préférence 2 à 45 %, de manière encore plus préférentielle 15 à 40 % du poids total de la composition.

13. Composition selon l'une quelconque des revendications précédentes, **caractérisée en ce que** la composition comprend au moins une charge choisie dans le groupe composé des nanocharges de silice, de la silice moléculaire, des nanocharges d'argile, des nanocharges de mica, des microfibres de polymères et des microfibres de verre.

14. Composition selon l'une quelconque des revendications précédentes, comprenant un extenseur de chaîne.

15. Composition selon l'une quelconque des revendications précédentes, **caractérisée en ce que** la taille moyenne en nombre des particules de charge est choisie dans la fourchette de 10 à 50 000 nm, de préférence de 10 à 1 000 nm et de manière encore plus préférentielle de 10 à 500 nm.

16. Kit d'éléments aptes à être mis en oeuvre lors d'une réparation vasculaire *in vivo*, comprenant une composition polymère biocompatible selon l'une quelconque des revendications 1 à 15, et une composition de catalyseur de durcissement, en vue du traitement prophylactique d'un os.

17. Kit selon la revendication 16, **caractérisé en ce que** la composition de catalyseur de durcissement comprend au moins un composant choisi dans le groupe composé des prépolymères matriciels, des charges et des agents de contraste.

18. Kit selon la revendication 16 ou la revendication 17, **caractérisé en ce que** la viscosité de la composition de catalyseur de durcissement est au maximum supérieure ou inférieure de 1 500 mm²/s [cSt] à la viscosité de la composition polymère biocompatible.

19. Kit selon l'une quelconque des revendications 16 à 18, **caractérisé en ce que** la composition polymère biocompatible mélangée à la composition de catalyseur de durcissement a un temps de durcissement de maximum 5 minutes, de préférence inférieur à 3 minutes.

20. Composition selon l'une quelconque des revendications 1 à 15, **caractérisée en ce que** l'os est une hanche ou la clavicule.
